# EUROPÄISCHE PATENTSCHRIFT

(11) **EP 0 781 291 B1**
(45) Veröffentlichungstag und Bekanntmachung des Hinweises auf die Patenterteilung: **08.12.2004**
(21) Anmeldenummer: 95908259.5
(22) Anmeldetag: 03.02.1995
(51) Int. Cl.: C07H 1/08

(54) **VERFAHREN ZUR ISOLIERUNG VON ZELLINHALTSSTOFFEN, WIE NUCLEINSÄUREN, AUS NATÜRLICHEN QUELLEN**
PROCESS FOR ISOLATING CELLULAR SUBSTANCES SUCH AS NUCLEIC ACIDS FROM NATURAL SOURCES
PROCEDE D'ISOLEMENT DE SUBSTANCES CONTENUES DANS DES CELLULES, TELLES QUE DES ACIDES NUCLEIQUES, A PARTIR DE SOURCES NATURELLES

(30) Priorität: 14.09.1994 DE 4432654
(43) Veröffentlichungstag der Anmeldung: 02.07.1997
(73) Patentinhaber: QIAGEN GmbH, 40724 Hilden (DE)
(72) Erfinder: COLPAN, Metin, D-45219 Essen (DE)
(74) Vertreter: Meyers, Hans-Wilhelm, Dr.Dipl.-Chem.
(86) Internationale Anmeldenummer: PCT/EP1995/000392
(87) Internationale Veröffentlichungsnummer: WO 1996/008500

(56) Entgegenhaltungen:
- EP-A- 0 268 946
- EP-A- 0 442 026
- DE-A- 4 139 664
- US-A- 4 923 978
- US-A- 5 004 806

## Beschreibung

Gegenstand der vorliegenden Erfindung ist ein Verfahren zur Präparation von Plasmid-DNA für die Gentherapie aus Mikroorganismen.

Bei der Präparation von Zellinhaltsstoffen, insbesondere von Nucleinsäuren, stellt sich oft das Problem, die aufgeschlossenen natürlichen Quellen, aus denen diese Inhaltsstoffe stammen, von den gelösten Stoffen zu trennen. Üblicherweise erfolgt die Abtrennung der Zellen oder Zelltrümmer mittels Zentrifugation, wobei sich die Zelltrümmer oder Zellen als Pellet im Zentrifugationsröhrchen abscheiden. Die gelösten Inhaltsstoffe finden sich dann im Überstand und können pipettiert werden. Bei der Präparation von Nucleinsäuren zur Abtrennung der aufgeschlossenen Zellen oder deren Bruchstücke konnten sich einfache Filtrationsverfahren nicht durchsetzen, da die Zelltrümmer entweder durch die zu grobporigen Filter durchlaufen und somit für Trübungen und Verunreinigungen im Filtrat sorgen oder bei Verwendung von Filtern mit entsprechend engen Poren es jedoch zwangsläufig zur Verstopfung der Filter kommt, so daß eine sinnvolle Präparation der Zellinhaltsstoffe nicht mehr möglich ist.

Der vorliegenden Erfindung liegt mithin das Problem zugrunde, ein Verfahren bereitzustellen und eine Vorrichtung zu schaffen, mit deren Hilfe Zentrifugationsschritte zur Präparation von Zellinhaltsstoffen aus natürlichen Quellen, wie Zellen, vermieden werden können durch einfacher zu handhabende Filtrationsschritte.

Das der Erfindung zugrundeliegende technische Problem wird gelöst durch ein Verfahren gemäß den Merkmalen des Anspruchs 1. Die sich daran anschließenden Unteransprüche betreffen bevorzugte Ausführungsformen des erfindungsgemäßen Verfahrens.

Um Inhaltsstoffe aus Zellen zu isolieren, werden diese üblicherweise zunächst aufgeschlossen. Bei der Präparation von Nucleinsäuren müssen die Zellen beispielsweise zunächst durch die Verwendung von Enzymen, wie z. B. Proteinase K, Lysozym und Detergenzien wie SDS, Brij, Triton X 100, Tween 10, DOC und Chemikalien wie Natriumhydroxid, Guanidin-Hydrochlorid und Guanidin-Isothiocyanat aufgeschlossen werden. Die Zellbruchstücke werden durch Zentrifugation sedimentiert und der Überstand dekantiert oder abpipettiert und anschließend durch eine Chromatographie oder Extraktion mit Phenol oder Chloroform und einer Alkoholfällung gereinigt. Maniatis; Current Protocols in Molecular Biology, Ausubel, F.M. et al., eds. (1991) Wiley Interscience, New York; Birnboim H.C. and Doly, J. (1979) A Rapid Alkaline Extraction Procedure for Screening Recombinant Plasmid DNA. Nucl. Acids Res. 7, pp 1513 - 1522.

Diese Zentrifugation in einer in Laboratorien üblichen Zentrifuge, z. B. Heraeus Biofuge GL, Beckmann GS6 dauert zwischen 15 min und 2 h bei 3.000 rpm bis 20.000 rpm, je nach Anwendungsfall und Partikelgröße der Zellbruchstücke. Daraus ergibt sich, daß bei großen Probenzahlen die Zentrifugation einen großen Personalaufwand und Zeitverluste bedeutet. Es ist daher wünschenswert, ein einfaches und schnelles Verfahren zur Entfernung der Zellbruchstücke zur Verfügung zu haben.

Die bisher verfügbaren Filtermaterialien und Filtrationsmethoden haben sich in Vorversuchen nicht zur Abtrennung dieser biologischen Zelltrümmer geeignet. Sterilfilter aus z. B. Nylon oder Celluloseacetat mit Porengrößen von 0,2 µm oder 0,45 µm verstopfen sofort und besitzen keine Kapazität, eine größere Menge an Zellbruchstücken zurückzuhalten. Diese Filter eignen sich lediglich zur Filtration von Flüssigkeiten mit einem nur sehr geringen Feststoff oder zellulären Kontaminationen.

Das erfindungsgemäße Filtrationsverfahren zur Präparation von plasmid-DNA für die Gentherapie aus Mikroorganismen geht davon aus, daß die Mikroorganismen mittels Enzymen, Chemikalien oder Detergenzien aufgeschlossen werden. Der Aufschluß wird für mindestens 1 minute ruhen gelassen. Der resultierende Aufschluß passiert dann eine Filterschicht aus geschüttetem Diol-Silicagel, Diol Diatomeenerde und/oder Diol-Perlite. Die aus der Filterschicht austretende Fraktion wird danach aufgefangen und anschließend die Nucleinsäure aus der aufgefangenen Fraktion isoliert und gereinigt.

Insbesondere mit einer geschütteten Filterschicht aus Silicagel mit einer Partikelgröße von 15 - 30 µm lassen sich Zellbruchstücke erfolgreich und ohne Verstopfen des Filters zurückhalten und es kann ein klares Lysat erhalten werden.

Die Filterschichten sind so modifiziert, daß keine Affinität zu Nucleinsäuren besteht.

In einer bevorzugten Ausführungsform kann der Fluß der Probe durch die Filterschicht durch Anlegen eines Überdrucks oder Unterdrucks erleichtert werden. Durch die Konfiguration der Porengröße der Filterschicht ist jedoch auch ein Durchgang der zu filtrierenden Probe durch die Filterschicht, getrieben durch die Schwerkraft, möglich. Desweiteren kann auch, zur Beschleunigung des Passierens der Probe durch die Filterschicht, die Probe durch Zentrifugation durch die Filterschicht befördert werden.

Die Filterschichten weisen Partikelgrößen zwischen 5 µm und 100 µm auf. Die besonders bevorzugte Filterschicht ist modifizierte Diol-Diatomeenerde mit Fließwerten von 0,1 bis 15 Darcy.

Das erfindungsgemäße Verfahren ist zur Präparation von Plasmid-DNA aus E. coli, Hefe oder eukaryontischen Zellen oder genomischer DNA aus Blut oder Zellen mit einer Größe von 1 bis 50 Kb geeignet. Das erfindungsgemäße Verfahren ist geeignet Plasmid-DNA zur Gentherapie, zu reinigen.

Der Überdruck auf der Seite vor der Passage durch die Filterschicht wird vorzugsweise mittels eines Kolben durchgeführt.

Vorzugsweise schließen sich an die Filtrierung weitere Aufarbeitungsschritte, wie Trennung an Anionenaustauschern und/oder Adsorption und Desorption von anderen mineralischen Trägern an.

Eine Vorrichtung zur Durchführung des Verfahrens besteht aus einem Hohlkörper, in dem die Filterschicht angeordnet ist. Die Filterschicht ist vorzugsweise zwischen zwei Fixierungseinrichtungen angeordnet. Als Hohlkörper kommt inbesondere ein Spritzenkörper in Betracht.

In einer bevorzugten Ausführungsform des erfindungsgemäßen Verfahrens werden gleichzeitig mehrere Proben aufgearbeitet und durch entsprechende Vorrichtungen, die in vorteilhafter Weise an Mikrotitrationssysteme adaptiert sind, geführt.

Eine geeignete Vorrichtung zur Durchführung des erfindungsgemäßen Verfahrens besteht aus einem vorzugsweise zylindrischen Hohlkörper mit Einlaß- und Auslaßöffnung sowie einer Filtrationseinrichtung, die im Hohlkörper angeordnet ist. Zur Fixierung der Filtrationseinrichtung können übliche Befestigungsmittel dienen, wie beispielsweise Verklebungen oder aber auch Fixierung durch Reibungskräfte, indem die Filtrationseinrichtung im Hohlkörper eingeklemmt ist.

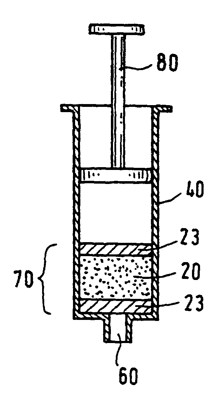

Die Vorrichtung besteht aus mindestens einer Filterschicht mit gleicher Porengröße in Richtung der Auslaßöffnung 60 gesehen. Die Figur 1 zeigt eine besonders bevorzugte Variante der Vorrichtung, indem im vorzugsweise zylindrischen Hohlkörper 40 die Filtrationseinrichtung 70 aus einer Schicht gestaltet ist. Die Partikelgröße der Filterschicht liegt im Bereich von 5 µm bis 500 µm bei einer gesamten Dicke der Filterschicht von 0,1 bis 200 mm.

Es kann vorteilhaft sein, eine zusätzliche Schicht 23 in dem Hohlkörper 40 anzuordnen, und zwar oberhalb und/oder unterhalb der Schicht 20, die ein vorzeitiges Eindringen der zu filtrierenden Lösung in den Filter oder das Austreten der Lösung aus der erfindungsgemäßen Vorrichtung verhindert.

Es ist jedoch möglich, die Schicht 20 als poröse, hydrophobe Schicht auszubilden. Ist die hydrophobe Trennschicht 23 oberhalb der Trennschicht 20 angeordnet, ist es vorteilhaft, wenn die Porengröße dieser Trennschicht nicht kleiner als diejenige der darunterliegenden Schicht 20 ist. Dieses Erfordernis ist bei der anderen Konfiguration, wobei die hydrophobe Trennschicht unterhalb der Schicht 20 angeordnet ist, nicht so kritisch.

Vorzugsweise weist die Vorrichtung einen Kolben 80 auf, über den ein Druck im Hohlkörper 40 aufgebaut werden kann, wodurch die Passage der Probe durch die Schicht 20 beschleunigt wird.

In einer weiteren bevorzugten Ausführungsform ist die erfindungsgemäße Vorrichtung mit weiteren zur Nucleinsäurepräparation notwe digen Instrumenten kombinierbar, wie sie z.B. in der DE-A- 41 39 664 offenbart sind.

Die DE-A-41 27 276 offenbart Anionenaustauscher, die in eine Membran eingebettet (3M Empore-Membran) sind. Diese Systeme sind unter der Bezeichnung QIAwell® handelsüblich.

### Beispiel 1

### Synthese von Diol-Diatomeenerde

100 g Diatomeenerde werden mit 2.000 ml 10 % Glymo (g-Glycido-Oxypropyltrimethoxsilan) in Toluol oder Tetrachlorkohlenstoff gemischt und entgast und 6 Stunden Rückfluß gekocht.

Die entstandene Epoxy-Diatomeenerde wird abgesaugt und mit Toluol und anschließend Methanol und Wasser gewaschen.

Zur Herstellung der Diol-Form wird die Epoxy-Diatomeenerde mit 10 mM H₂SO₄/Wasser 3 h unter Rückfluß gekocht.

Die entstandene Diol-Diatomeenerde wird gründlich mit Wasser gewaschen und getrocknet.

Die getrocknete Diol-Diatomeenerde wird direkt zur Filtration eingesetzt. Die Diol-Diatomeenerde kann durch andere Synthesevorschriften, wie sie in der Literatur beschrieben werden, synthetisiert werden.

### Beispiel 2

### Filter für Fermenter Kulturen

Eine ca. 2.000 ml Chromatographiesäule (10cm x 25cm) wird am unteren Ende mit einer passenden 50 µm PE-Fritte oder einem Nylon-Netz verschlossen und 5 cm hoch mit Diol-Diatomeenerde gefüllt. Die Diol-Diatomeenerde wird mit einem sehr groben Filtertuch (ca. 150 - 200 µm) abgedeckt. 2 Liter E. coli Zell-Lysat (Hergestellt aus Lyse von 1 vol. E. coli Zellen in TE mit 1 vol. 0,1 M NaOH/1 % SDS und Neutralisation mit 1 vol. 3M Kalium-Acetat, pH 4.5) werden in die Filtersäule gefüllt und 15 Min. ruhen gelassen. Das Filtrat wird durch die untere Säulenöffnung mit einer Schlauchpumpe abgepumpt und direkt durch eine Anionenaustauscher-Chromatographiesäule mit DEAE-Sepharose FF (Pharmacia) geleitet.

### Beispiel 3 (nicht erfindungsgemäß)

Eine 15 ml PE-Einmalspritze (Durchmesser: 1,5 x 15 cm) wird nach Abbildung 1 unten mit einer porösen 50 µm PE-Fritte verschlossen und 1 cm hoch mit 15 - 25 µm Silicagel gefüllt und oben wiederum mit einer 50 µm Fritte verschlossen. Eine 100 ml E.coli Zellkultur wird abzentrifugiert und in 5 ml TE-Puffer (10 mM Tris, 1 mM EDTA, pH 7.5) resuspendiert und durch Zugabe von 1 Vol. 0,1 M NaOH / 1 % SDS lysiert. Die Zell-Lyse wird durch Zugabe von 1 Vol. 3 M Kaliumacetat, pH 4,8 gestoppt und das Zell-Lysat inklusive der ausgefallenen Proteine, K-Dodecylsulfat, genomische DNA und Zellbruchstücke in die Filtersäule gefüllt. Das Zell-Lysat ruht 10 min, wobei die leichteren Zellbruchstücke, Proteine und das ausgefallene KDS nach oben steigen. Diese Inkubation verhindert ein vorschnelles Verstopfen der Filterschicht mit den Zellbruchstücken und erhöht somit die Schmutzaufnahme und Rückhaltekapazität der Filterschicht um das Mehrfache. Mit einem Kolben wird das Filtrat durch die Silicagelschicht gedrückt. Das klare Filtrat kann anschließend sofort einer weiteren Nucleinsäurereinigung über Anionenaustauscher-Chromatographie, Gelfiltration oder einer Fällung mit Alkohol unterzogen werden.

Die Filterschicht mit den Zellbruchstücken wird verworfen.

## Patentansprüche

1. Filtrationsverfahren zur Präparation von Plasmid-DNA für die Gentherapie aus Mikroorganismen, wobei
• die Mikroorganismen mittels Enzymen, Chemikalien oder Detergenzien aufgeschlossen werden,
• der Aufschluss für mindestens eine Minute ruht,
• der resultierende Aufschluss eine Filterschicht aus geschüttetem Diol-Silicagel, Diol-Diatomeenerde und/oder Diol-Perlite passiert,
• die aus der Filterschicht austretende Fraktion aufgefangen wird und
• anschließend die Plasmid-DNA aus der aufgefangenen Fraktion isoliert und gereinigt wird.

2. Verfahren nach Anspruch 1, wobei die Filterschicht aus Diol-Diatomeenerde mit Fließwerten von 0,1 bis 15 Darcy besteht.

3. Verfahren nach mindestens einem der Ansprüche 1 und/oder 2, wobei die Filterschicht in einem Hohlkörper zwischen zwei Fixierungseinrichtungen angeordnet ist.

4. Verfahren nach mindestens einem der Ansprüche 1 bis 3, wobei die Filterschicht in einem Spritzenkörper zwischen zwei Glas- oder Kunststofffritten angeordnet ist.

5. Verfahren nach einem der Ansprüche 1 bis 4, wobei ein Überdruck auf der Seite vor der Passage durch die Filterschicht mittels eines Kolben oder ein Unterdruck auf der Seite der Filterschicht, die nach der Passage liegt ausgeübt wird.

## Claims

1. A filtration process for the preparation of plasmid DNA for gene therapy from microorganisms, wherein
• the microorganisms are digested by enzymes, chemicals or detergents,
• the digest rests for at least one minute,
• the resulting digest passes through a filter layer of poured diol-silicagel, diol-diatomaceous earth and/or diol-perlite,
• the fraction flowing out of the filter layer is collected and
• subsequently the plasmid DNA is isolated from the collected fraction and purified.

2. The process according to claim 1, wherein the filter layer consists of diol-diatomaceous earth having flow values from 0.1 to 15 Darcy.

3. The process according to at least one of claims 1 and/or 2, wherein the filter layer is located in a hollow body between two fixing devices.

4. The process according to at least one of claims 1 to 3, wherein the filter layer is located in a syringe body between two glass or plastic frits.

5. The process according to any one of claims 1 to 4, wherein a piston exerts an overpressure on the side upstream of the passage through the filter layer or a reduced pressure is exerted on the side of the filter layer downstream of the passage.

## Revendications

1. Procédé de filtration pour la préparation d'ADN plasmidique destiné à la thérapie génique à partir de microorganismes, dans lequel
- les microorganismes sont attaqués à l'aide d'enzymes, de produits chimiques ou de détergents,
- on laisse reposer le produit obtenu pendant au moins une minute,
- on fait passer le produit résultant à travers une couche filtrante constituée de diol-gel de silice, de diol-terre de diatomées et/ou de diol-perlite,
- la fraction sortant de la couche filtrante est recueillie et
- ensuite, l'ADN plasmidique est isolé à partir de la fraction recueillie, et purifié.

2. Procédé selon la revendication 1, dans lequel la couche filtrante est constituée de diol-terre de diatomées ayant un coefficient d'écoulement compris entre 0,1 et 15 darcy.

3. Procédé selon au moins une des revendications 1 et/ou 2, dans lequel la couche filtrante est disposée dans un corps creux entre deux dispositifs de fixation.

4. Procédé selon au moins une des revendications 1 à 3, dans lequel la couche filtrante est disposée dans un corps de seringue entre deux couches de fritte de verre ou de matière plastique.

5. Procédé selon une des revendications 1 à 4, dans lequel une pression est exercée en amont du passage à travers la couche filtrante à l'aide d'un piston ou une dépression est exercée du côté de la couche filtrante qui est en aval du passage.
